# FASCICULE DE BREVET EUROPEEN

(11) **EP 0 748 200 B1**
(45) Date de publication et mention de la délivrance du brevet: **10.06.1998**
(21) Numéro de dépôt: 95911361.4
(22) Date de dépôt: 01.03.1995
(51) Int. Cl.: A61J 1/20, A61J 1/06

(54) **ENSEMBLE POUR LA REPARTITION D'UNE SOLUTION PHARMACEUTIQUE DANS DES CARPULES OU ANALOGUES, NOTAMMENT APRES REPRISE D'UN PRODUIT LYOPHILISE**
VORRICHTUNG ZUM VERTEILEN VON PHARMAZEUTISCHEN LÖSUNGEN IN AMPULLEN ODER DERGL.INSBESONDERE NACH MISCHUNG MIT EINER LYOPHILISIERTEN LÖSUNG
UNIT FOR DISTRIBUTING A PHARMACEUTICAL SOLUTION IN AMPOULES OR THE LIKE, IN PARTICULAR AFTER RECOVERY OF A LYOPHILIZED PRODUCT

(30) Priorité: 04.03.1994 FR 9402526
(43) Date de publication de la demande: 18.12.1996
(73) Titulaire: PASTEUR MERIEUX SERUMS ET VACCINS, 69007 Lyon (FR)
(72) Inventeur: GENET, Alain, F-69130 Ecully (FR)
(74) Mandataire: Colombet, Alain André
(86) Numéro de dépôt international: FR9500240
(87) Numéro de publication internationale: WO9523576

(56) Documents cités:
- EP-A- 0 327 519
- WO-A-92/15347
- US-A- 3 564 256
- US-A- 4 507 113
- US-A- 4 842 028

## Description

La présente invention a trait à un ensemble pour la répartition d'une solution pharmaceutique, contenue dans un flacon multi-doses, dans des carpules mono-doses à usage unique ou analogues et plus particulièrement pour la mise en solution d'un produit pharmaceutique lyophilisé contenu dans un tel flacon et la répartition de la solution pharmaceutique obtenue. L'invention s'applique de manière particulièrement avantageuse dans le cas des vaccins destinés à être conservés sous forme lyophilisée jusqu'à leur utilisation et qui nécessitent donc une présentation séparée du principe actif lyophilisé et du solvant. Elle s'applique préférentiellement à l'injection par jet sous pression (injection sans aiguille).

L'invention a également trait à des ensembles de carpules mono-doses sous forme de grappes, à des pistons perfectionnés pour carpules, à un appareil spécialement adapté à la mise en solution de vaccins lyophilisés et à la répartition en carpules, ainsi qu'à un procédé pour ce faire.

On utilise de plus en plus, pour l'administration de substances médicamenteuses par voie parentérale, et notamment pour la vaccination, des appareils d'injection sans aiguille délivrant la dose sous forme d'un jet très mince et très puissant. Ces applications se sont développées, non seulement en médecine vétérinaire, mais également en médecine humaine, l'absence d'aiguille d'injection permettant d'utiliser un personnel formé rapidement, d'augmenter les cadences et d'éviter les contaminations bactériennes et surtout virales qui peuvent se produire dans le cas où une même buse d'injection est utilisée sans stérilisation entre deux ou plusieurs sujets, ce qui est la pratique courante.

Les plus perfectionnés des appareils à injection par jet transcutané utilisent des carpules à usage unique munies de leur propre buse, laquelle buse doit être la seule partie en contact avec la peau du patient lors de l'injection, ce qui supprime le risque de contamination croisée, étant donné que l'on change la carpule et donc la buse à chaque injection.

Toutefois, ce type d'appareil n'a pu être utilisé jusqu'à présent que pour des vaccins prêts à l'emploi, conditionnés au préalable en doses uniques dans des carpules mono-doses (uni-doses). De telles carpules sont par exemple connues sous la marque Imule.

Le problème reste entier dans le cas des produits pharmaceutiques dont la conservation n'est garantie que sous une forme lyophilisée et qui doivent donc être remis en solution extemporanément. Cela est particulièrement vrai pour les vaccins, dont beaucoup doivent être conservés sous forme lyophilisée.

Leur conditionnement nécessite donc de disposer d'un flacon de solvant et d'un flacon de produit actif lyophilisé. On utilise alors une seringue de grand volume avec laquelle on récupère le solvant, puis on injecte ce solvant dans le facon de produit lyophilisé. Ensuite, après agitation, on peut reprendre la solution pharmaceutique prête à l'emploi à l'aide d'autant de seringues à usage unique que de doses contenues dans le flacon de produit lyophilisé.

Or, on se rend compte que, dans la pratique, en plus des risques de contamination croisée lorsqu'une même aiguille est utilisée sans stérilisation pour traiter plusieurs sujets, existe également un risque de contamination important lors des opérations de remise en solution des produits lyophilisés.

D'autres inconvénients majeurs sont l'imprécision des doses administrées et le fait que l'on arrive difficilement à réaliser le nombre de doses qui est prévu pour un flacon de vaccin donné. En effet, même pour des personnes expérimentées, on n'est pas certain de pouvoir administrer le nombre de doses prévu, à moins de prévoir un excédent de vaccin, ce qui entraîne alors un coût plus élevé. On notera notamment la perte de produit lors de l'opération qui consiste à chasser l'air contenu dans la seringue avant l'administration.

La demande de brevet européen EP-A-0 327 519 décrit un dispositif mélangeur permettant la mise en solution d'un produit lyophilisé, puis son aspiration dans un corps de seringue. Ce dispositif est conçu pour être mis en oeuvre en une seule opération et pour protéger l'utilisateur contre tout risque de contact avec le produit solubilisé. Pour ce faire, le dispositif comprend essentiellement trois réceptables, un pour un flacon de produit lyophilisé, un pour un flacon souple de solvant du produit lyophilisé et un pour un corps de seringue, ainsi qu'un système de valve. Le dispositif est conçu pour permettre le passage, par simple gravité, du solvant dans le flacon de produit lyophilisé, le mélange et la solubilisation de ce produit. Le produit solubilisé peut ensuite être soit aspiré directement par la seringue soit d'abord aspiré dans le flacon souple, après avoir exercé une pression sur ce flacon qui joue le rôle de pompe, puis aspiré à l'aide de la seringue.

La présente invention a ainsi pour objectif de fournir un ensemble permettant en particulier la mise en solution de produits pharmaceutiques lyophilisés et sa répartition en carpules mono-doses à usage unique ou analogues dans des conditions optimales de sécurité, notamment de stérilité, et de rendement, tout en permettant d'utiliser les flacons habituels de produits pharmaceutiques lyophilisés.

Elle a également pour objectif de fournir un tel ensemble qui présente un faible encombrement, qui soit aisé à utiliser en toute sécurité et qui soit peu coûteux et fiable afin d'en permettre une utilisation à grande échelle dans les meilleures conditions notamment dans les pays dans lesquels on souhaite procéder à des traitements ou des vaccinations de masse.

Elle a encore pour objectif de fournir un tel ensemble qui soit en même temps utilisable pour la simple répartition d'une solution prête à l'emploi dans de telles carpules.

La présente invention a pour objet un ensemble pour la répartition d'une solution pharmaceutique, contenue dans un flacon multi-doses, dans des carpules mono-doses à usage unique ou dispositifs analogues, en particulier pour la mise en solution d'un produit pharmaceutique lyophilisé contenu dans un tel flacon et la répartition de la solution pharmaceutique obtenue, comprenant des carpules mono-doses à usage unique et un appareil de répartition, ensemble dans lequel sont prévus des moyens de refoulement/aspiration conçus pour pouvoir faire circuler un liquide des carpules vers le flacon et du flacon vers les carpules, par l'intermédiaire d'un réseau de canaux appropriés, et, éventuellement un flacon de produit pharmaceutique liquide ou lyophilisé.

De préférence, il est prévu des moyens de réglage du volume de solution pharmaceutique envoyé dans les carpules. Par réglage, il faut entendre la répartition homogène dans les carpules en évitant toute entrée d'air dans celles-ci et avantageusement aussi le réglage de la quantité de solution admise dans les carpules. En effet, dans le cas des produits lyophilisés, on préfère que le solvant soit contenu dans les carpules et, pour tenir compte des pertes de solvant du fait du volume mort et éviter toute entrée d'air dans les carpules, on préfère que celles-ci contiennent au départ un volume un peu supérieur au volume de reprise. En d'autres termes, après reprise, on admet dans les carpules un volume un peu inférieur au volume initial.

De préférence, les carpules mono-doses sont formées d'un corps cylindrique délimitant un volume intérieur dans lequel coulisse, de façon étanche, un piston conçu pour faire varier le volume intérieur, les pistons des carpules mono-doses servant de moyens de refoulement/aspiration pour faire circuler le liquide des carpules vers le flacon et vice versa. De préférence, le corps cylindrique des carpules mono-doses est fermé à une extrémité par un col présentant un orifice central formant buse pour jet sous pression, le piston étant aussi conçu pour transmettre au liquide la pression d'un injecteur et ainsi assurer la formation de ce jet.

Dans le mode de réalisation préféré de l'invention, les carpules sont présentées par groupes de plusieurs en étant montées de manière étanche et amovible sur une pièce de support qui présente intérieurement un canal central desservant, par des canaux collatéraux, chacune des carpules mono-doses du groupe, le canal central débouchant à une extrémité de la pièce de support qui est agencée de manière que le canal central puisse être raccordé au volume intérieur d'un flacon multi-doses. Cette présentation permet de travailler de façon très aisée et rapide.

De préférence, l'appareil de répartition comporte un logement dans lequel viennent s'adapter, de manière amovible, les carpules mono-doses (éventuellement la pièce de support réunissant les carpules), des tiges de poussée agencées chacune de manière à pouvoir venir en prise amovible avec le piston d'une carpule et des moyens d'actionnement pour déplacer les tiges de poussée, et partant les pistons des carpules, dans le sens de la réduction du volume intérieur de celles-ci ou dans le sens inverse, des moyens étant en outre prévus pour assurer, dans ce dernier cas, la libération de la prise entre pistons et tiges de poussée, et en même temps le réglage du volume admis dans chaque carpule, de telle manière que chaque carpule contienne alors le volume requis de solution pharmaceutique. Cet agencement simple et sûr est très avantageux, puisqu'il permet d'aspirer la solution dans les carpules en même temps et à la même vitesse et, lorsque le volume requis a été aspiré, de libérer les carpules alors prêtes à l'emploi. Il permet très avantageusement aussi d'éviter toute introduction d'air dans les carpules.

Les moyens de libération de la prise entre piston et tige de poussée peuvent être avantageusement des pièces annulaires calibrées portées par l'appareil et dont le diamètre extérieur est légèrement plus petit que le diamètre intérieur du corps de carpule, les pièces annulaires étant conçues pour se trouver a l'intérieur d'un corps de carpule de manière à former une butée pour le piston lors de son déplacement dans le sens de l'augmentation du volume intérieur de la carpule.

La combinaison des moyens de prise entre tiges de poussée et piston et des moyens du type pièce annulaire permet d'assurer parfaitement les fonctions attendues, cela d'une manière remarquablement simple et fiable, sans recouvrir à des moyens mécaniques, hydrauliques, et/ou de commande complexe. Cette simplicité permet justement de recourir aux grappes de carpules avantageuses de l'invention.

La pièce de support peut présenter des carpules sur une face ou sur deux faces opposées. On préfère la présentation sur une seule et même face.

De préférence, chaque tige de poussée est munie à une extrémité d'un moyen de prise complémentaire d'un moyen de prise présenté par les pistons des carpules.

L'ensemble selon l'invention pourra incorporer les grappes de carpules et/ou pièces de support et/ou appareils de répartition tels que décrits ci-après.

L'invention a également pour objet une grappe de carpules mono-doses à usage unique destinées à l'injection par jet sous pression d'une solution pharmaceutique et réunies sur une pièce de support. La pièce de support comporte de préférence intérieurement un réseau de canaux agencés de manière à desservir les carpules. De préférence, les carpules peuvent comprendre une extrémité présentant un fin orifice et formant buse d'injection, tandis que la pièce de support peut comprendre des réceptacles, dans lesquels les extrémités de carpule formant buse d'injection sont placées de façon étanche et amovible et que le réseau de canaux de la pièce de support peu comprendre, chaque fois, un canal collatéral débouchant dans le réceptacle au regard du fin orifice de la buse d'injection de la carpule en place.

Dans un mode de réalisation préféré de l'invention, la pièce de support comporte les carpules sur une même face. De préférence alors, la pièce de support, de forme allongée, peut comporter un canal central dans lequel est inséré un axe mobile en rotation dans ce canal, cet axe étant muni d'une rainure s'étendant le long de l'axe et sur une partie de la longueur de celui-ci, la rainure étant agencée de manière à pouvoir mettre en communication les carpules ou à interrompre cette communication, et d'une gorge, tandis que la pièce de support comporte un orifice de sortie débouchant dans le canal central au regard de la gorge. De préférence, les carpules disposées sur une rangée.

Ce mode de réalisation présente l'avantage d'isoler chaque carpule individuellement en supprimant toute communication entre les carpules. Cela permet d'utiliser les carpules de manière étalée dans le temps sans risque de contamination.

Pour assurer une étanchéité appropriée, on préfère utiliser une pièce de support en matériau souple, par exemple caoutchouc ou tout matériau élastique accepté par la pharmacopée. On peut alors lui adjoindre un renfort pour le regidifier.

Dans un deuxième mode de réalisation, la pièce de support peut aussi avoir une forme allongée avec deux rangées de carpules disposées dans un même plan, de part et d'autre de la pièce de support. Bien entendu, elle peut avoir toute autre forme appropriée et même porter plus de deux rangées de carpules, ou encore n'en porter qu'une seule.

De préférence, dans ce deuxième mode de réalisation, la pièce de support comporte intérieurement un canal central débouchant à une extrémité et duquel partent des canaux collatéraux. La pièce de support peut être avantageusement formée d'un seul tenant en matière plastique moulée, ce qui permet d'obtenir des coûts de production très favorables et compatibles avec l'usage unique auquel est destiné cette pièce.

Les pièces de support selon l'invention peuvent être agencées de manière à pouvoir être reliées à une aiguille usuelle destinée à traverser le bouchon du flacon de produit pharmaceutique lyophilisé afin d'assurer l'entrée du solvant dans celui-ci, puis son retrait après reconstitution de la solution pharmaceutique dans le cas des produits lyophilisés.

De préférence, les carpules comprennent un corps cylindrique délimitant un volume intérieur et un piston adapté pour se déplacer de façon étanche dans ledit corps pour faire varier le volume intérieur, celui-ci, lorsque le piston est dans sa position rétractée maximale, étant supérieur d'une valeur réglée au volume de dose auquel est destiné la carpule, ce qui permet de tenir compte des pertes inévitables de solvant sur les parois du flacon de produit pharmaceutique lyophilisé et dans l'aiguille et les canaux de la pièce de support (volume mort), et d'éviter toute entrée d'air dans les carpules.

Les carpules peuvent comprendre très avantageusement un piston ayant une face extérieure munie en son centre d'un évidemment allant s'agrandissant vers le milieu du piston.

L'invention a aussi pour objet une pièce de support servant à réunir des carpules mono-doses à usage unique ou analogues et à assurer un écoulement de liquide de et vers ces carpules, comportant un corps allongé muni d'un canal central et de canaux collatéraux débouchant à l'extérieur et dans ce canal, canal central dans lequel est inséré un axe mobile en rotation dans ce canal, cet axe comportant une rainure s'étendant le long de l'axe et sur une partie de la longueur de celui-ci et agencée de manière à pouvoir venir en regard des canaux collatéraux pour leur mise en communication, et une gorge, tandis que la pièce de support comporte un orifice de sortie débouchant dans le canal central au regard de la gorge. De préférence, le corps allongé est en matière souple, caoutchouc ou autre matière élastique acceptée par la pharmacopée et peut être associé à un renfort rigide. De préférence encore, le corps allongé comporte des réceptacles dans lesquels débouchent les canaux collatéraux et qui sont adaptés aux carpules.

L'invention a également pour objet un piston pour carpule destinée à l'injection sous pression d'une solution pharmaceutique, qui soit spécialement adapté aux carpules selon l'invention et à l'utilisation qui en est faite et qui est décrite ci-dessus. Ces pistons se caractérisent dans le fait qu'ils comportent, sur leur face extérieure par rapport au reste de la carpule, un moyen de prise par blocage de forme, qui peut comprendre avantageusement un évidemment central allant s'agrandissant vers le milieu du piston.

L'invention a aussi pour objet les carpules pour injection par jet sous pression, qui sont munies d'un tel piston.

L'invention a également pour objet un appareil de répartition d'une solution pharmaceutique, contenue dans un flacon multi-doses, dans des carpules mono-doses à usage unique ou analogues, en particulier pour la mise en solution d'un produit pharmaceutique lyophilisé et la répartition de la solution pharmaceutique obtenue, comprenant un logement délimité sur au moins un côté par une structure fixe ou mobile dans le plan du logement et par des tiges de poussée ou moyens analogues qui traversent la structure par des perçages équidistants appropriés et qui sont mobiles dans le plan du logement par coulissement dans les perçages.

De préférence, à son extrémité située du côté du logement, chaque tige de poussée comporte un moyen de prise pour blocage de forme, celui-ci pouvant comprendre avantageusement un téton muni d'une tête extrême reliée à la tige de poussée par une partie de plus petite section.

A chaque perçage de la structure, il est de préférence associé un rebord annulaire calibré qui fait saillie à l'intérieur du logement.

Les tiges de poussée peuvent être portées par des mâchoires extérieures mobiles dans le plan du logement. De préférence encore, si les structures mobiles ou mâchoires intérieures peuvent être normalement sollicitées l'une vers l'autre par des ressorts de rappel, les mâchoires extérieures seront déplaçables en translation dans un sens et dans l'autre par un moyen d'actionnement qui pourra être manuel ou motorisé. De préférence, chaque mâchoire extérieure est munie d'un moyen de butée maintenant la mâchoire intérieure associée en position ouverte contre l'action de son ressort de rappel.

L'appareil pourra être conçu pour une grappe de carpules dans laquelle les carpules sont disposées sur un seul côté de la pièce de support, ce qui est le cas préféré, ou sur deux côtés. Dans ce dernier cas, on prévoit deux jeux de mâchoires mobiles en translation dans le plan du logement des carpules, un jeu intérieur de mâchoires délimitant le logement et présentant des perçages équidistants et un jeu extérieur de mâchoires portant les tiges de poussée, celles-ci étant guidées en translation dans lesdits perçages et les pièces annulaires étant des rebords annulaires qui sont portés par les mâchoires intérieures et bordent le débouché des perçages du côté du logement.

Enfin, l'appareil selon l'invention peut comporter un support pour recevoir un flacon multi-doses, ce support pouvant être avantageusement un support réglable destiné à recevoir des flacons de dimensions variées.

L'invention a également encore pour objet un procédé pour la mise en solution d'un produit pharmaceutique lyophilisé contenu dans un flacon multi-doses et sa répartition en carpules mono-doses à usage unique, notamment, pour injection par jet sous pression, dans lequel on utilise des carpules mono-doses contenant le solvant adapté au produit lyophilisé, on fait passer simultanément le solvant contenu dans l'ensemble des carpules dans le flacon de produit lyophilisé, on agite de manière à bien solubiliser le produit, puis on répartit, de manière dosée, la solution pharmaceutique ainsi produite simultanément dans l'ensemble des carpules mono-doses.

De préférence, on choisit au départ des carpules mono-doses contenant du solvant en excès pour tenir compte des pertes de solvant au cours du procédé.

L'invention va être maintenant décrite plus en détail à l'aide de deux exemples de réalisation de grappes de carpules et d'appareils conformes à l'invention, en liaison avec le dessin annexé dans lequel :
- la figure 1 représente une vue en coupe longitudinale médiane d'un ensemble comprenant dix carpules sur leur pièce de support, d'une aiguille et d'un flacon de vaccin lyophilisé ;
- la figure 2 représente une vue en coupe médiane d'un piston de carpule conforme à l'invention ;
- la figure 3 représente une vue de côté d'un appareil conforme à l'invention sur lequel l'ensemble de la figure 1 est monté, l'appareil étant représenté sans les mâchoires intérieures et extérieures ;
- la figure 4 est une vue en plan de la représentation de la figure 3, l'appareil étant ici représenté avec ses mâchoires ;
- les figures 5 à 7 sont des vues partielles en coupe transversale destinées à montrer le mode de fonctionnement de l'appareil des figures 3 et 4 ;
- la figure 8 représente une vue de face d'une pièce de support selon un deuxième exemple de réalisation ;
- la figure 9 représente une coupe longitudinale selon VIII-VIII de la pièce de support de la figure 8,
- la figure 10 est une vue en élévation d'une autre pièce de ce deuxième exemple de réalisation, à plus grande échelle ;
- la figure 11 est une vue en coupe selon X-X de la pièce représentée à la figure 1 ;
- la figure 12 est une vue en élévation d'une grappe de carpules conforme au deuxième exemple de réalisation ;
- les figures 13 et 14 sont une vue schématique expliquant le fonctionnement du deuxième exemple de réalisation.

La carpule 1 conforme à l'invention est réalisée en polypropylène. Elle comporte un corps cylindrique 2 ouvert à son extrémité postérieure 3 et refermé à son extrémité antérieure 4 par un fond 5 qui se poursuit par une partie rétrécie ou col 6 qui présente la buse d'injection 7, finement calibrée, qui débouche dans le volume intérieur 8 du corps 2. 0,60 ml de solvant pour vaccin lyophilisé est contenu dans le volume 8 refermé à son extrémité postérieure par un piston 9 assurant une étanchéité parfaite et réalisé d'un seul tenant en matériau élastomère.

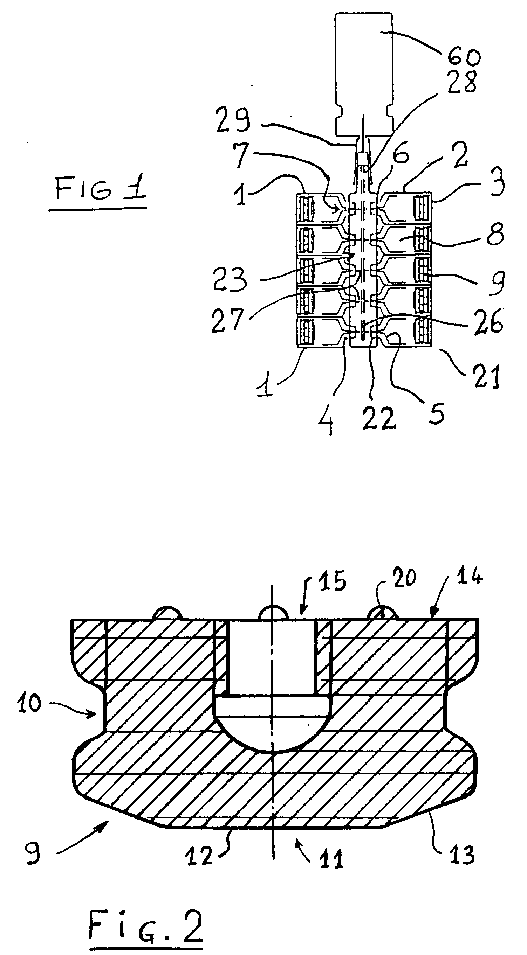

Le piston 9 est représenté en détail et à grande échelle à la figure 2, où l'on voit sa forme essentiellement cylindrique présentant un évidemment circonférentiel 10 dont les bords s'écartent d'environ 30° par rapport à un plan transversal à la génératrice du cylindre. La face d'attaque 11 du piston 9 présente une partie centrale 12 coïncidant avec la base du cylindre et une partie périphérique 13 légèrement inclinée Cette forme permet à cette face du piston de venir épouser le fond 5 lorsque le piston a été actionné.

La face opposée du piston 9, qui est la face située à l'extérieur par rapport au volume 8 et est désignée par le repère 14, est essentiellement plane, mais présente un évidemment central 15 s'étendant, centré sur l'axe, par une première partie cylindrique 16, une deuxième partie cylindrique 17 de diamètre un peu supérieur, formant ainsi un épaulement 18, puis par une partie en forme de calotte sphérique 19 dont la base a le même diamètre que la partie 17. La face 14 présente encore quatre picots 20 équidistants entre eux et aussi vis-à-vis de l'axe du cylindre, qui interviennent dans la fabrication des pistons notamment pour leur démoulage.

On revient à la figure 1 où l'on voit une grappe 21 de dix carpules 1 identiques, du type Imule ^{R} , chacune fixée de manière amovible mais étanche par son col 6 dans un réceptacle approprié 22 porté par la pièce repérée 23. Cette pièce, de forme allongée, présente, à cet effet, une série de cinq réceptacles 22 sur ses deux faces opposées 24 et 25, et un fin canal central 26 desservant chacun des réceptacles 22 en leur centre, par des canaux collatéraux 27 débouchant en regard des buses d'injection 7. Le canal central 26 débouche à une extrémité 28 de la pièce 23, laquelle extrémité 28 est conçue pour que vienne s'adápter sur elle une aiguille 29 d'un type usuel. La pièce 23 est réalisée en matière plastique et est obtenue, avec son canal central et ses canaux collatéraux, d'une seule pièce par moulage. Lorsqu'elles sont convenablement montées sur la pièce 23, les carpules 1 sont bord à bord.

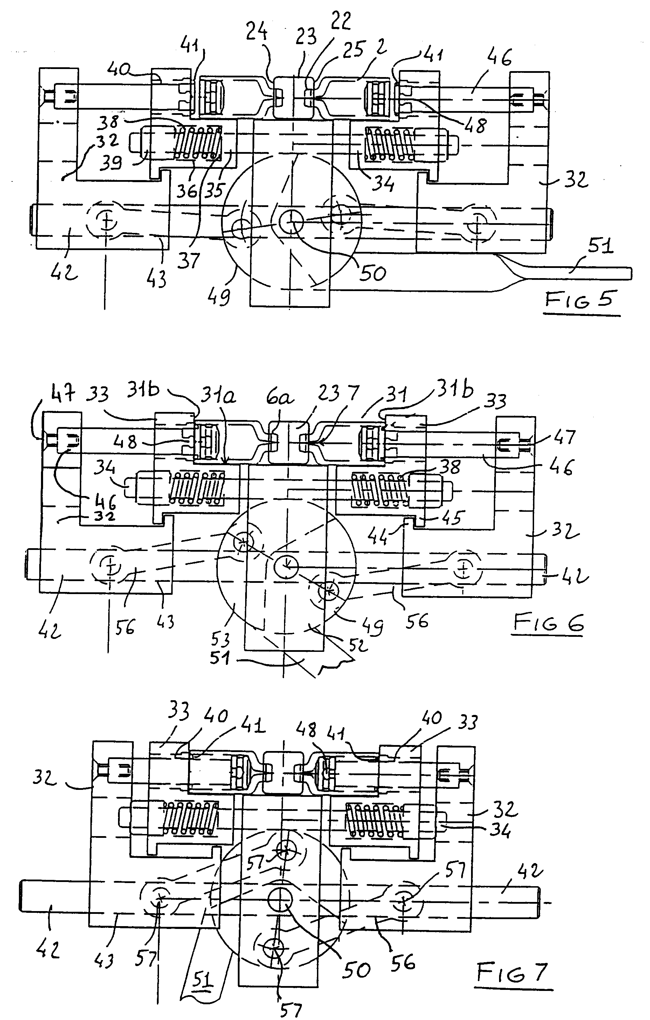

On notera que les figures 5 à 7 montrent des carpules 1 dont le col 6 présente en sus un petit rebord 6a destiné, si nécessaire, à améliorer la tenue des carpules 1 vis-à-vis de la pièce 23.

Pour son application préférée aux vaccins lyophilisés, on conditionne stérilement la grappe 21 avec ses dix carpules 1 remplies du solvant approprié (0,6 ml par carpule pour des carpules destinées à contenir 0,5 ml de vaccin en solution).

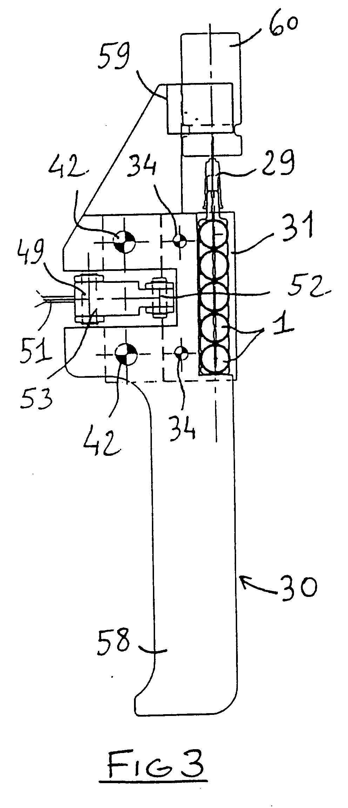

On se réfère maintenant aux figures 3 à 7 relatives à un appareil 30 destiné à recevoir la grappe 21 de carpules et le flacon de vaccin lyophilisé 31 pour assurer l'ensemble des opérations de préparation du vaccin prêt à l'emploi et sa distribution précise dans les carpules.

L'appareil 30 comprend d'abord un logement 31 pour recevoir une grappe 21 de carpules de manière centrée. Il comprend aussi deux jeux de mâchoires, des mâchoires extérieures 32 et des mâchoires intérieures 33, ces dernières ayant une forme générale en L et venant en regard l'une de l'autre de manière à délimiter le logement 31, comme on peut le voir plus particulièrement aux figures 5 à 7, la base des L formant le fond 31a du logement et les branches du L, les bords 31b. Les mâchoires intérieures 33 sont montées mobiles en translation sur deux axes 34 parallèles au plan du logement 31, et donc à celui de la grappe 21 de carpules lorsque celle-ci y est en place.

Ces axes 34 s'étendent dans des perçages 35 pratiqués dans les mâchoires intérieures 33, dans la base du L qui forme le fond du logement 31, ces perçages 35 présentant une partie 36 de plus grandes dimensions (en largeur ou en diamètre), ce qui procure des épaulements 37. Des ressorts de rappel 38 hélicoïdaux s'étendent concentriquement aux axes 34 dans la partie 36 des perçages 35, entre les extrémités des axes 34, où ils sont maintenus par des boulons 39, et les épaulements 37. Ces ressorts 38 sollicitent donc chacune des mâchoires intérieures 33 en direction l'une de l'autre, c'est-à-dire en direction de la grappe 21 montée dans le logement 31. Les boulons 39 peuvent permettre le réglage de la pression d'application des ressorts 38. Un moyen de blocage de sûreté peut leur être associé.

Les branches du L formant les bords 31b du logement 31 présentent chacune cinq perçages 40 équidistants et agencés de manière à venir se placer exactement au regard de l'extrémité postérieure 3 d'une carpule 1 de la grappe 21 en place. Le diamètre des perçages 40 est inférieur à celui des pistons 9 des carpules 1. A chaque perçage 40 est associé un petit rebord annulaire 41 qui fait saillie à l'intérieur du logement 31. Le diamètre intérieur de ce rebord 41 est identique au diamètre intérieur des perçages 40, tandis que son diamètre extérieur est sensiblement identique à celui du piston 9 des carpules 1, ce qui signifie qu'il est adapté pour pouvoir pénétrer à l'intérieur de la carpule 1 par l'extrémité postérieure de celle-ci en poussant le piston 9.

Les mâchoires extérieures 32 de l'appareil 30 ont également une forme de L et sont montées coulissantes sur deux axes parallètes 42 s'inscrivant dans un plan parallèle au plan des axes 34 des mâchoires intérieures 33.

Les axes 42 traversent les bases du L par des perçages 43 et les mâchoires 32 sont empêchées de s'échapper grâce à des épaulements 44 venant en appui contre des épaulements 45 correspondants portés par les mâchoires intérieures 33. A chaque branche du L sont fixées cinq tiges de poussée 46 équidistantes. Une partie de chaque tige de poussée pénètre dans le corps de la branche du L, dans un perçage approprié, pour y être fixé à l'aide d'une vis 47. Les tiges de poussée 46 s'étendent ensuite au travers des perçages 40 pratiqués dans les mâchoires intérieures 33 dans lesquels elles peuvent coulisser. A leur extrémité libre, les tiges de poussée 46 présentent un téton 48 ayant une forme complémentaire de l'évidemment central 15 des pistons 9, ce qui signifie que ces tétons 48 peuvent venir en prise amovible dans ces évidemments.

Comme on peut le voir aux figures 5 à 7, le rebord annulaire 41 de la mâchoire intérieure 33 peut faire partie d'une pièce unique formant en même temps la chemise dans laquelle coulisse la tige de poussée 46 associée et qui est placée dans un perçage 40 approprié.

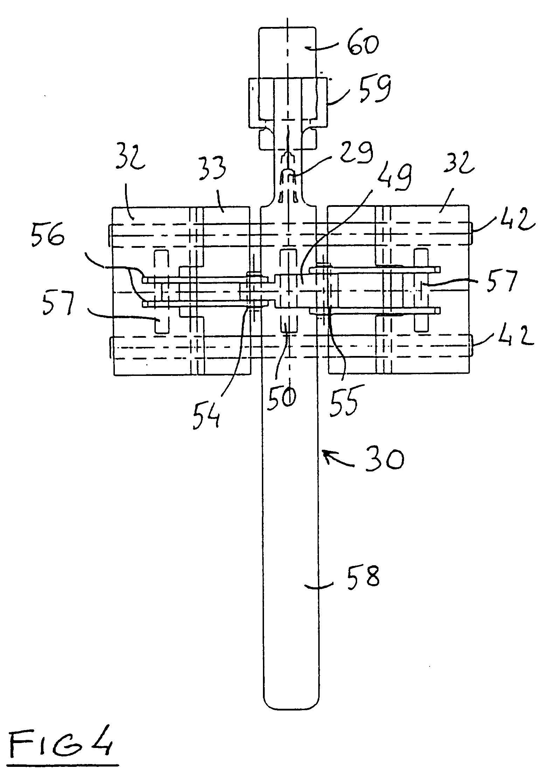

Les mâchoires extérieures 32 sont actionnées dans le sens de leur déplacement en translation par rapport aux axes 42, par un dispositif d'actionnement comprenant un plateau-manivelle 49 tourillonné sur l'appareil 30 sur un axe 50 et dont on voit le profil aux figures 3 et 4 et la forme en plan aux figures 5 à 7. Une manette 51 est solidaire du plateau-manivelle 49 et sert à l'actionnement de ce dernier. Le plateau-manivelle 49 se présente sous forme d'un disque ayant une partie 52 de faible épaisseur et une partie 53 plus épaisse. Chacune de ces parties 52 et 53 présente un perçage, respectivement 54 et 55, les axes de ces perçages et l'axe 50 étant alignés et les perçages étant situés chacun à une même distance de l'axe 50.

Comme on le voit bien à la figure 4, deux doubles biellettes 56 sont tourillonnées par rapport aux perçages 54 et 55 et, à leurs autres extrémités, sur les mâchoires extérieures 32 par l'intermédiaire d'axes 57 parallèles entre eux et par rapport à l'axe 50.

On voit enfin que l'appareil 30 comporte encore une poignée 58 et, à l'opposé de celle-ci par rapport à l'ensemble mécanique qui vient d'être décrit, un support 59 sur lequel on monte un flacon 60 de vaccin lyophilisé.

Pour le fonctionnement de l'appareil, on se reportera maintenant aux figures 5 à 7.

La figure 5 représente la première étape où l'on place un ensemble constitué d'une grappe 21 de carpules, d'une aiguille 29 et d'un flacon 60 (tel qu'on peut le voir à la figure 1), dans le logement 31 de l'appareil 30. A ce moment, la manette 51 est parallèle aux axes des mâchoires et les doubles biellettes parallèles entre elles mais non alignées. Les mâchoires intérieures sont écartées de la grappe 21, étant empêchées de s'en rapprocher par le jeu des épaulements 44 et 45.

A la figure 6, la manoeuvre convenable de la manette 51 provoque le déplacement des mâchoires extérieures 32, ce qui permet aux mâchoires intérieures 33 de venir en appui contre les extrémités postérieures des carpules 1, sous l'action des ressorts de rappel 38. Les rebords annulaires 41 sont alors positionnés en partie à l'intérieur même des carpules 1 si la position du piston est initialement un peu rentrée par rapport à l'extrémité postérieure 3 de la carpule, comme on le voit sur les figures.

Le positionnement d'une grappe de carpules peut très avantageusement être assuré par les mâchoires intérieures sous l'action de leurs ressorts de rappel et par les rebords annulaires pénétrant légèrement dans les carpules.

A la figure 7, la poursuite de la manoeuvre de la manette 51 provoque le déplacement des mâchoires extérieures 32. De ce fait, les tiges de poussée 46 coulissent dans les perçages 40 et les pistons sont poussés jusqu'à venir en butée sur le fond 5 des carpules 1. Les tétons 48 ont pénétré dans les évidements centraux 15 des pistons et les rebords annulaires 41 sont positionnés à l'intérieur des carpules. Le solvant contenu dans les carpules 1 se retrouve dans le flacon de vaccin lyophilisé après être passé dans les canaux 27 et 26 de la pièce 23.

En tenant l'appareil par la poignée 58, on secoue l'ensemble de manière à bien solubiliser le vaccin lyophilisé. Ensuite, on ramène progressivement la manette 51 vers sa position initiale de la figure 5. La prise des tétons 48 dans les évidemments centraux 15 des pistons 9 assure le déplacement de ces derniers, et le vaccin prêt à l'emploi est ainsi aspiré jusque dans les carpules 1. On comprend que la présence des rebords annulaires 41 procure un effet de butée permettant le dosage de reprise du liquide et empêchant les pistons 9 de venir en alignement avec les extrémités postérieures 3 des carpules 1. Les rebords annulaires 41 sont conformés très avantageusement de manière que le volume intérieur 8, initialement de 0,6 ml, soit limité par le piston 9 à 0,5 ml, ce qui est la dose recherchée et évite, étant donné la perte obligatoire de solvant sur les parois du flacon dans l'aiguille et dans les canaux, toute entrée d'air dans les carpules.

Lorsque les pistons sont en butée contre les rebords annulaires, la poursuite de la manoeuvre de la manette 51 assure le décrochement des tétons 48 et le retour à la position de la figure 5.

On comprend bien entendu que l'on pourrait aussi utiliser l'appareil 30 ainsi décrit pour effectuer, sur le terrain, la répartition en carpules d'un vaccin liquide prêt à l'emploi contenu dans un flacon, en utilisant une grappe de carpules vides conditionnées stérilement (carpules vides ou remplies d'air ou de gaz stérile). Dans ce cas, le support 59 pourrait avantageusement être réglable pour lui permettre d'accueillir des flacons de plus grandes dimensions à partir desquels l'on pourrait préparer plusieurs grappes de carpules.

De même, l'invention n'est pas limitée au mode de réalisation décrit ci-dessus. L'appareil et la grappe pourraient être prévus pour un nombre différent de carpules, par exemple 20. On peut également concevoir qu'un appareil donné puisse être utilisé avec une grappe ayant un nombre de carpules plus réduit que les grappes auxquelles cet appareil est principalement destiné. Dans ce cas aussi, un support réglable peut être prévu. La réalisation d'un tel support est bien sûr à la portée de l'homme du métier et n'a donc pas à être décrite en détail ici. Pour des raisons d'encombrement, on pourrait par exemple préférer utiliser à cinq reprises l'appareil décrit ci-dessus dans le cas des flacons de 50 doses de vaccin lyophilisé que l'on trouve couramment.

Dans un mode de réalisation perfectionné, la manette est remplacée par un moteur électrique, fonctionnant de préférence sur accumulateurs. De même, l'ensemble du mécanisme d'actionnement des mâchoires pourrait être réalisé différemment sans remettre en cause le principe de l'invention.

L'invention a été décrite à propos de carpules, mais on comprend qu'elle est applicable à des dispositifS analogues, par exemple des seringues mono-doses comprenant un corps, un piston et un embout pour recevoir une aiguille d'injection, et contenant une préparation lyophilisée, le corps et le piston coopérant avec les autres moyens de l'invention comme décrit pour les carpules.

En conséquence, on entend par "carpule", dans les présentes, non seulement les carpules proprement dites mais également des dispositifs analogues.

On va maintenant décrire un mode de réalisation préféré de l'invention.

A la différence du précédent, il prévoit d'utiliser un agencement linéaire des carpules.

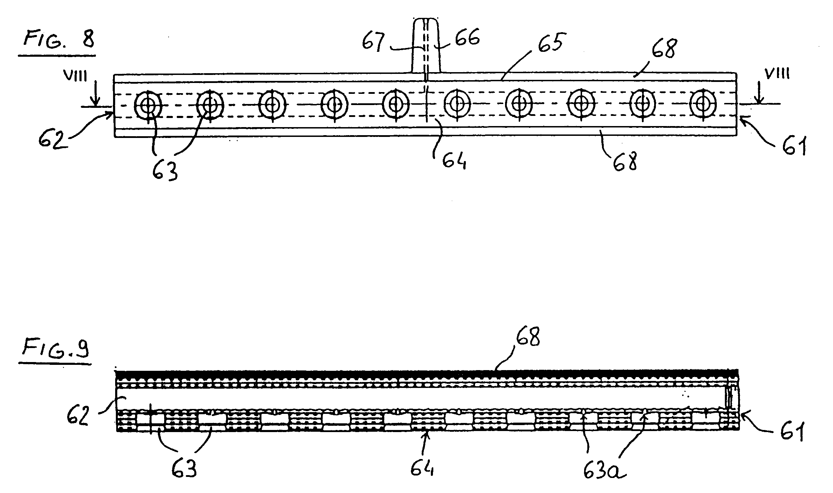

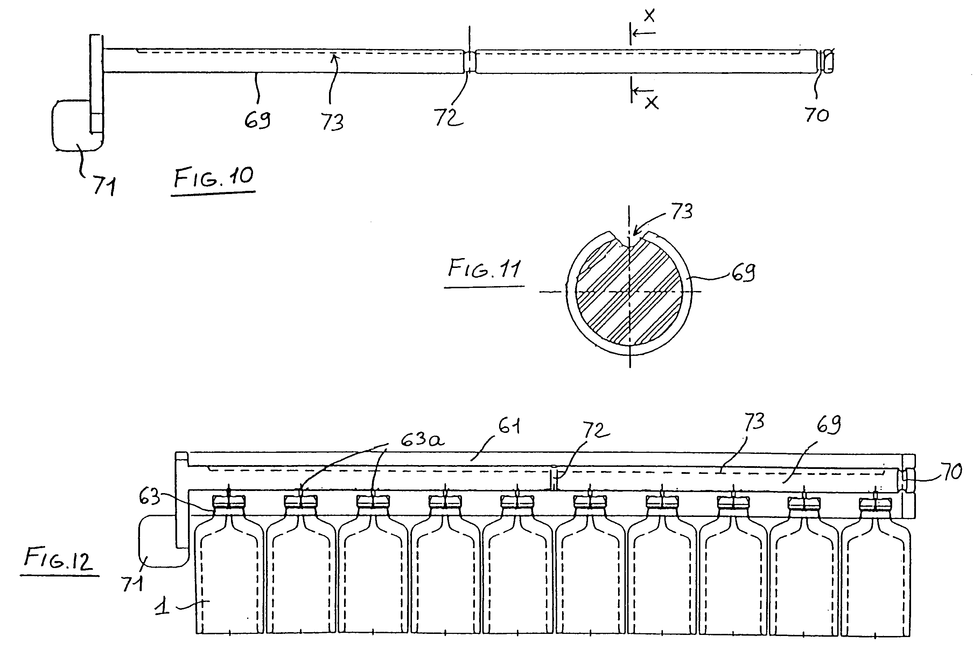

A la place de la pièce 23 du premier exemple de réalisation, on utilise une pièce allongée 61 (figures 8 et 9) de section carrée ou angulaire, comportant un canal central 62 débouchant aux deux extrémités et un nombre prédéterminé (10 dans le mode de réalisation représenté) d'orifices 63 formant réceptacles, répartis de façon équidistante sur une même face 64 de la pièce allongée 61, ces orifices 63 communiquant avec le canal central 62 par un fin canal 63a et présentant un profil complémentaire de celui de la tête des carpules, avec de préférence des parties en relief complémentaires pour assurer un bon maintien des carpules en place. On notera que ces carpules diffèrent simplement des précédentes par leur tête qui présente un relief.

Sur une face 65 orthogonale à la face 64, la pièce allongée 61 comporte une partie en saillie 66 munie d'un fin canal 67 raccordé au canal central 62. Cette partie en saillie forme un embout 66 destiné à recevoir une aiguille usuelle telle que 29 à la figure 1 (corps plus aiguille proprement dite).

La pièce allongée 61 avec ses différentes parties qui viennent d'être décrites est formée, notamment moulée, d'un seul tenant en matière souple, telle que caoutchouc ou autre matière élastique acceptée par la pharmacopée, par exemple en Santoprene.

Afin de rigidifier cette pièce allongée 61, on lui adjoint un renfort 68, par exemple en matière plastique rigide, telle que Polypropylène, surmoulé ou rapporté. Le renfort 68 représenté est un profité en U laissant libre la face 64 et un passage pour l'embout 66.

Le canal central 62, qui peut avoir par exemple un diamètre de l'ordre de 4 mm, est destiné à recevoir à son intérieur un axe rigide 69 (figure 10), de préférence en matière plastique et avantageusement dans la même matière que les carpules et le renfort. L'axe 69 est formé d'une tige cylindrique présentant au voisinage d'une extrémité une gorge 70, à l'extrémité opposée une poignée 71, au centre un amincissement circonférentiel ou gorge centrale 72. Comme on le voit en coupe à la figure 11 et en trait interrompu aux figures 8 et 10, l'axe 69 comporte en outre une rainure 73 s'étendant sur presque toute la longueur de l'axe.

On se réfère maintenant à la figure 12, où l'on voit l'ensemble monté. L'axe 69 a été mis en place dans le canal central 62 dans lequel il est étroitement adapté et maintenu par un blocage de forme entre la gorge 70 et une partie complémentaire venue du moulage avec la pièce allongée 61. La gorge centrale 72 se trouve dans l'axe de l'embout 66 de manière à communiquer avec le canal 67 de celui-ci. Des carpules 1 sont en place, maintenues par leur tête dans les orifices 63 formant réceptacle. Dans la position représentée sur cette figure, la rainure 73 se trouve à l'opposé des orifices 63 et des fins canaux 63a, de sorte que les carpules sont isolées entre elles et de l'extérieur. Seule une rotation de 180° de l'axe 69 au moyen de la poignée 71 permet de mettre en communication l'ensemble des fins de canaux 63a avec la rainure 73 et partant, avec la gorge centrale 72 et le canal 67 de l'embout 66.

La souplesse du matériau constituant la pièce allongée 61 permet d'assurer, au contact de l'axe 69 et des carpules, une étanchéité appropriée vis-à-vis de l'extérieur et entre les différents orifices et carpules entre eux.

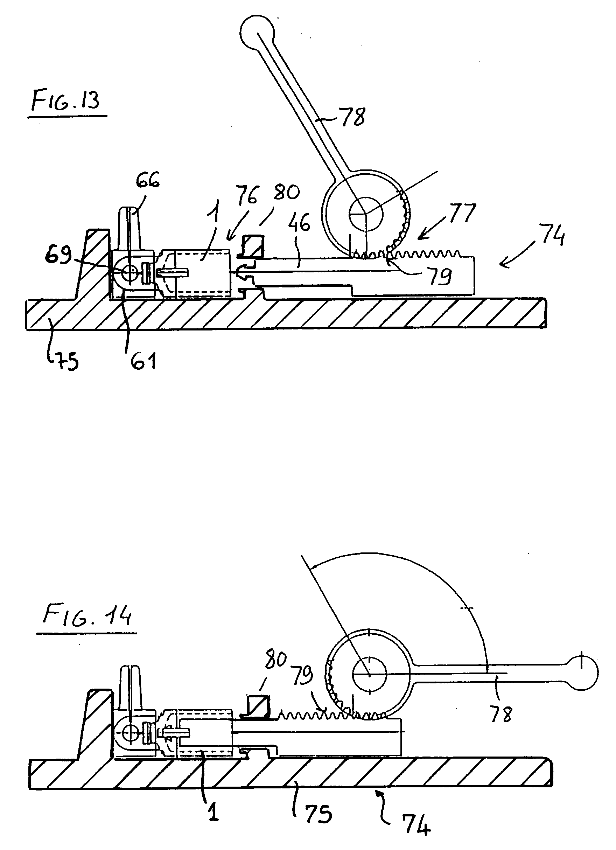

On se réfère maintenant aux figures 13 et 14 où se trouve représenté schématiquement l'appareil de répartition 74 qui comprend un plateau de support 75 muni d'un logement 76 recevant l'ensemble monté qui vient d'être décrit. Le plateau s'utilise avantageusement en étant placé horizontalement sur une table avec les carpules horizontales et l'embout dirigé verticalement et relié, par l'intermédiaire d'une aiguille 29 non représentée, à un flacon de produit à solubiliser (également non représenté).

Sur le plateau de support 75 est prévu un dispositif d'actionnement 77 comprenant une manette 78 et des moyens 79 pour faire se déplacer de façon rectiligne des tiges de poussée 46 analogues pour l'essentiel à celles du précédent mode de réalisation, de manière à pouvoir actionner les pistons des carpules 1 dans le sens de refoulement et dans le sens de l'aspiration.

Le fonctionnement est aisé à comprendre. Après mise en place de l'ensemble pièce allongée 61 - carpules 1 comprenant un solvant - axe 69 - aiguille 29 - flacon de produit à solubiliser, on tourne l'axe 69 de 180° pour mettre en communication les carpules 1 et le flacon, puis on actionne les moyens 79 de manière que les tiges de poussée 46 poussent sur les pistons des carpules 1 jusqu'à ce que le solvant contenu dans ces dernières soit passé dans le flacon. Après solubilisation, il ne reste plus qu'à actionner les moyens 79 en sens inverse pour aspirer la préparation prête à l'emploi dans les carpules (à noter que les tiges de poussée 46 et pistons des carpules coopèrent comme décrit au regard du premier mode de réalisation). On ramène alors l'axe 69 dans sa position initiale, si bien que les carpules remplies sont isolées les unes des autres et peuvent être utilisées de manière espacée dans le temps.

Les moyens 79 n'ont pas été décrits en détail. Toutefois, l'homme du métier est tout à fait en mesure de concevoir et réaliser différentes solutions pour actionner les tiges de poussée de la manière indiquée.

Bien entendu, certaines particularités décrites en détail dans le premier exemple de réalisation peuvent être reprises dans celui-ci, notamment les moyens de réglage du volume aspiré dans les carpules et les moyens de libération entre pistons et tiges de poussée.

## Revendications

1. Ensemble pour la répartition d'une solution pharmaceutique, contenue dans un flacon multi-doses, dans des carpules mono-doses à usage unique, en particulier pour la mise en solution d'un produit pharmaceutique lyophilisé contenu dans un tel flacon et la répartition de la solution pharmaceutique obtenue, comprenant des carpules mono-doses (1) et un appareil (30 ; 74) de répartition, ensemble dans lequel sont prévus des moyens (9, 46) de refoulement/aspiration conçus pour pouvoir faire circuler un liquide des carpules (1) vers le flacon (60) et du flacon (60) vers les carpules (1), par l'intermédiaire d'un réseau de canaux (26, 27 ; 62, 67, 72, 73) approprié, et, éventuellement, un flacon (60) de produit pharmaceutique liquide ou lyophilisé.

2. Ensemble selon la revendication 1, caractérisé en ce qu'il comprend des moyens de réglage du volume de solution pharmaceutique envoyé dans les carpules (1).

3. Ensemble selon la revendication 1 ou 2, caractérisé en ce que les carpules mono-doses (1) sont formées d'un corps cylindrique (2) délimitant un volume intérieur (8) dans lequel coulisse, de façon étanche, un piston (9) conçu pour faire varier le volume intérieur (8), les pistons (9) des carpules mono-doses (1) servant de moyens de refoulement/aspiration pour faire circuler le liquide des carpules (1) vers le flacon (60) et vice versa.

4. Ensemble selon la revendication 3, caractérisé en ce que le corps cylindrique (2) des carpules mono-doses (1) est fermé à une extrémité (4) par un col (6) présentant un orifice central (7) formant buse pour injection par jet sous pression, le piston (9) étant aussi conçu pour assurer la formation de ce jet.

5. Ensemble selon l'une quelconque des revendications 1 à 4, caractérisé en ce que les carpules (1) sont représentées par groupe de plusieurs en étant montées de manière étanche et amovible sur une pièce de support (23 ; 61) qui présente intérieurement un canal central (26 ; 62) desservant, par des canaux collatéraux (27 ; 63a), chacune des carpules mono-doses (1) du groupe, le canal central (26 ; 62) débouchant par une partie (28 ; 66), de la pièce de support (23 ; 61), qui est agencée de manière que le canal central (26 ; 62) puisse être raccordé au volume intérieur d'un flacon multi-doses (60).

6. Ensemble selon l'une quelconque des revendications 2 à 5, caractérisé en ce que l'appareil (30, ; 74) de répartition comporte un logement (31 ; 76) dans lequel viennent s'adapter de manière amovible les carpules mono-doses (1), des tiges de poussée (46) agencées chacune de manière à pouvoir venir en prise amovible avec le piston (9) d'une carpule (1) et des moyens d'actionnement (49, 51 ; 77, 78) pour déplacer les tiges de poussée (46) et, partant, les pistons (9) des carpules (1) dans le sens de la réduction du volume intérieur (8) de celles-ci ou dans le sens inverse, des moyens (41) étant en outre prévus pour assurer, dans ce dernier cas, la libération de la prise entre pistons (9) et tiges de poussée (46), de telle manière que chaque carpule (1) contienne alors le volume requis de solution pharmaceutique.

7. Ensemble selon la revendicaton 6, caractérisé en ce que les moyens de libération de la prise entre pistons (9) et tiges de poussée (46) sont des pièces annulaires calibrées (41) portées par l'appareil (30 ; 74) et dont le diamètre extérieur est légèrement plus petit que le diamètre intérieur du corps (2) de carpule (1), les pièces annulaires (41) étant conçues pour se trouver à l'intérieur d'un corps (2) de carpule de manière à former une butée pour le piston (9) de la carpule (1) lors de son déplacement dans le sens de l'augmentation du volume intérieur (8) de la carpule (1).

8. Ensemble selon l'une des revendications 5 à 7, caractérisé en ce que la pièce de support (61) présente les carpules (1) sur une seule et même face (64).

9. Ensemble selon l'une des revendications 5 à 7, caractérisé en ce que la pièce de support (23) présente les carpules (1) sur deux faces opposées.

10. Ensemble selon l'une quelconque des revendications 6 à 9, caractérisé en ce que chaque tige de poussée (46) est munie à une extrémité d'un moyen de prise (48) complémentaire d'un moyen de prise (15) présenté par les pistons (9) des carpules (1).

11. Ensemble selon l'une quelconque des revendications 1 à 10, caractérisé en ce qu'il comprend une grappe de carpules (1) selon l'une quelconque des revendications 12 à 21, et/ou une pièce de support selon l'une quelconque des revendications 22 à 24 et/ou un appareil de répartition selon l'une quelconque des revendications 25 à 28.

12. Grappe de carpules mono-doses (1) à usage unique, destinées à l'injection d'une solution pharmaceutique par jet sous pression et réunies sur une pièce de support (23 ; 61) qui comporte intérieurement un réseau de canaux (26, 27 ; 62, 63a, 67, 72, 73) agencé de manière à desservir les carpules (1).

13. Grappe de carpules (1) selon la revendication 12, caractérisée en ce que les carpules (1) comprennent une extrémité (4) présentant un fin orifice et formant une buse d'injection(7), en ce que la pièce de support (23 ; 61) comprend des réceptacles (22 ; 63) dans lesquels les extrémités de carpule formant buse d'injection (7) sont placées de manière étanche et amovible et en ce que le réseau de canaux de la pièce de support (23 ; 61) comprend chaque fois un canal collatéral (27 ; 63a) débouchant dans le réceptacle (22 ; 63) au regard du fin orifice de la buse d'injection (7) de la carpule (1) en place.

14. Grappe de carpules (1) selon la revendication 12 ou 13, caractérisée en ce que la pièce de support (61) comporte les carpules (1) sur une même face (64).

15. Grappe de carpules (1) selon la revendication 14, caractérisée en ce que la pièce de support (61) comporte un canal central (62) dans lequel est inséré un axe (69) mobile en rotation dans ce canal, cet axe (69) étant muni d'une rainure (73) s'étendant le long de l'axe (69) et sur une partie de la longueur de celui-ci, la rainure (73) étant agencée de manière à pouvoir mettre en communication les carpules (1) ou à interrompre cette communication, et d'une gorge (72), tandis que la pièce de support (61) comporte un orifice de sortie (67) débouchant dans le canal central (62) au regard de la gorge (72).

16. Grappe de carpules (1) selon la revendication 15, caractérisée en ce que les carpules (1) sont disposées sur une rangée.

17. Grappe de carpules (1) selon l'une quelconque des revendications 14 à 16, caractérisée en ce que la pièce de support (61) est en matière souple et est associée à un renfort rigide (68).

18. Grappe (21) de carpules (1), selon la revendication 12 ou 13, caractérisée en ce que la pièce de support (23) a une forme allongée et comporte deux rangées de carpules (1) disposées dans un plan, de part et d'autre de la pièce de support (23).

19. Grappe de carpules (1) selon l'une quelconque des revendications 12 à 18, caractérisée en ce que la pièce de support (23 ; 61) est agencée de manière à pouvoir être reliée à une aiguille usuelle (29).

20. Grappe de carpules (1) selon l'une quelconque des revendications 12 à 19, caractérisée en ce que les carpules (1) comprennent un corps cylindrique (2) délimitant un volume intérieur (8) et un piston (9) adapté pour se déplacer de façon étanche dans ledit corps (2), pour faire varier le volume intérieur (8) et en ce que, lorsque le piston (9) est dans sa position rétractée maximale, le volume intérieur (8) est supérieur d'une valeur réglée au volume de dose auquel est destinée la carpule (1).

21. Pièce de suppport servant à réunir des carpules mono-doses à usage unique et à assurer un écoulement de liquide de et vers ces carpules, comprenant un corps allongé (61) muni d'un canal central (62) et de canaux collatéraux (63a) débouchant à l'extérieur et dans le canal central, canal central (62) dans lequel est inséré un axe (69) mobile en rotation dans ce canal, cet axe (69) comportant une rainure (73) s'étendant le long de l'axe (69) et sur une partie de la longueur de celui-ci et agencée de manière à pouvoir venir au regard des canaux collatéraux (63a) pour leur mise en communication, et une gorge (72), tandis que la pièce de support (61) comporte un orifice de sortie (67) débouchant dans le canal central (62) au regard de la gorge (72).

22. Pièce de suppport selon la revendication 21, caractérisée en ce que le corps allongé (61) est en matière plastique souple et associé à un renfort rigide (68).

23. Pièce de support selon la revendication 21 ou 22, caractérisée en ce que le corps allongé (61) comprend des réceptacles dans lesquels débouchent les canaux collatéraux et qui sont adaptés aux carpules (1).

24. Appareil de répartition d'une solution pharmaceutique, contenue dans un flacon multi-doses, dans des carpules mono-doses à usage unique, en particulier pour la mise en solution d'un produit pharmaceutique lyophilisé et la répartition de la solution pharmaceutique obtenue, comprenant un logement (31 ; 76) délimité sur au moins un côté par une structure (33 ; 80) fixe ou mobile dans le plan du logement (31) et par des tiges de poussée (46) qui traversent la structure (33 ; 80) par des perçages (40) équidistants appropriés et sont mobiles dans le plan du logement (31 ; 76) par coulissement dans les perçages (40).

25. Appareil selon la revendication 24, caractérisé en ce que, à son extrémité située du côté du logement (31 ; 76), chaque tige de poussée (46) comporte un moyen de prise (48) par blocage de forme.

26. Appareil selon la revendication 25, caractérisé en ce que le moyen de prise comprend un téton (48) muni d'une tête extrême reliée à la tige de poussée (46) par une partie de plus petite section.

27. Appareil selon l'une quelconque des revendications 24 à 26, caractérisé en ce que, à chaque perçage (40) de la structure (33 ; 80), il est associé un rebord annulaire (41) calibré qui fait saillie à l'intérieur du logement (31).

28. Procédé pour la mise en solution d'un produit pharmaceutique lyophilisé contenu dans un flacon multi-doses et sa répartition en carpules mono-doses à usage unique, notamment pour injection par jet souspression, dans lequel on utilise des carpules mono-doses contenant le solvant adapté au produit lyophilisé et reliées au flacon multi-doses par un réseau de canaux, on fait passer simultanément le solvant contenu dans l'ensemble des carpules dans le flacon de produit lyophilisé, on agite de manière à bien solubiliser le produit, puis on répartit de manière dosée la solution pharmaceutique ainsi produite simultanément dans l'ensemble des carpules mono-doses, solvant et solution pharmaceutique circulant dans le réseau de canaux par refoulement et aspiration.

29. Procédé selon la revendication 28, caractérisé en ce que l'on choisit au départ des carpules mono-doses contenant du solvant en excès pour tenir compte des pertes de solvant au cours de procédé.

## Claims

1. Unit for distributing a pharmaceutical solution, contained in a multi-dose bottle, into single-dose ampoules for a single use, in particular for dissolving a lyophilised pharmaceutical product contained in a bottle of this type and the distribution of the pharmaceutical solution obtained, comprising single-dose ampoules (1) and a distribution apparatus (30; 74), in which unit delivery/suction means (9, 46) are provided, which are designed in order to be able to circulate a liquid from the ampoules (1) towards the bottle (60) and from the bottle (60) towards the ampoules (1), through the intermediary of an appropriate network of channels (26, 27; 62, 67, 72, 73), and, possibly, a bottle (60) of liquid or lyophilised pharmaceutical product.

2. Unit according to Claim 1, characterised in that it comprises means for regulating the volume of pharmaceutical solution sent into the ampoules (1).

3. Unit according to Claim 1 or 2, characterised in that the single-dose ampoules (1) are formed by a cylindrical body (2) defining an inner volume (8) in which there slides, in a tight manner, a piston (9) designed for varying the inner volume (8), the pistons (9) of the single-dose ampoules (1) serving as delivery/suction means for circulating the liquid from the ampoules (1) to the bottle (60) and vice versa.

4. Unit according to Claim 3, characterised in that the cylindrical body (2) of the single-dose ampoules (1) is closed at one end (4) by a neck (6) having a central orifice (7) forming a nozzle for injection by a jet under pressure, the piston (9) also being designed in order to ensure the formation of this jet.

5. Unit according to one of Claims 1 to 4, characterised in that the ampoules (1) are represented by a group of several whilst being mounted in a liquid-tight and detachable manner on a support member (23; 61) which internally comprises a central channel (26; 62) supplying, by collateral channels (27; 63a), each of the single-dose ampoules (1) of the group, the central channel (26; 62) passing out through a part (28; 66) of the support member (23; 61), which is arranged in order that the central channel (26; 62) may be connected to the inner volume of a multi-dose bottle (60).

6. Unit according to one of Claims 2 to 5, characterised in that the distribution apparatus (30; 74) comprises a housing (31; 76) in which are detachably fitted the single-dose ampoules (1), pushing rods (46) each arranged in order to be able to come into detachable engagement with the piston (9) of an ampoule (1) and actuation means (49, 51; 77, 78) for moving the pushing rods (46) and, consequently, the pistons (9) of the ampoules (1) in the direction of a reduction of the inner volume (8) of the latter or in the opposite direction, means (41) furthermore being provided for ensuring, in this latter case, the release of the engagement between the pistons (9) and pushing rods (46), such that each ampoule (1) then contains the required volume of pharmaceutical solution.

7. Unit according to Claim 6, characterised in that the means for releasing the engagement between the pistons (9) and pushing rods (46) are calibrated annular members (41) supported by the apparatus (30; 74) and whereof the outer diameter is slightly smaller than the inner diameter of the body (2) of the ampoule (1), the annular members (41) being designed in order to be located inside an ampoule body (2) in order to form an abutment for the piston (9) of the ampoule (1) at the time of its displacement in the direction of increasing the inner volume (8) of the ampoule (1).

8. Unit according to one of Claims 5 to 7, characterised in that the support member (61) comprises the ampoules (1) on one and the same side (64).

9. Unit according to one of Claims 5 to 7, characterised in that the support member (23) comprises ampoules (1) on two opposite sides.

10. Unit according to one of Claims 6 to 9, characterised in that each pushing rod (46) is provided at one end with engagement means (48) complementing engagement means (15) presented by the pistons (9) of the ampoules (1).

11. Unit according to one of Claims 1 to 10, characterised in that it comprises a cluster of ampoules (1) according to one of Claims 12 to 21 and/or a support member according to one of Claims 22 to 24 and/or a distribution apparatus according to one of Claims 25 to 28.

12. Cluster of single-dose ampoules (1) for single use, intended for the injection of a pharmaceutical solution by a pressurised jet and which are combined on a support member (23; 61) which comprises internally a network of channels (26, 27; 62, 63a, 67, 72, 73) arranged in order to serve the ampoules (1).

13. Cluster of ampoules (1) according to Claim 12, characterised in that the ampoules (1) comprise one end (4) having a fine orifice and forming an injection nozzle (7), in that the support member (23; 61) comprises receptacles (22; 63) in which the ends of the ampoule forming an injection nozzle (7) are placed in a liquid-tight and detachable manner and in that the network of channels of the support member (23; 61) comprises each time a collateral channel (27; 63a) opening into the receptacle (22; 63) opposite the fine orifice of the injection nozzle (7) of the ampoule (1) in place.

14. Cluster of ampoules (1) according to Claim 12 or 13, characterised in that the support member (61) comprises the ampoules (1) on the same side (64).

15. Cluster of ampoules (1) according to Claim 14, characterised in that the support member (61) comprises a central channel (62) in which is inserted a spindle (69) able to rotate in this channel, this spindle (69) being provided with a groove (73) extending along the spindle (69) and over part of the length of the latter, the groove (73) being arranged in order to be able to connect the ampoules (1) or to interrupt this connection, and with a groove (72), whereas the support member (61) comprises an outlet orifice (67) opening into the central channel (62) facing the groove (72).

16. Cluster of ampoules (1) according to Claim 15, characterised in that the ampoules (1) are arranged in a row.

17. Cluster of ampoules (1) according to one of Claims 14 to 16, characterised in that the support member (61) is made of flexible material and is associated with a rigid reinforcement (68).

18. Cluster (21) of ampoules (1), according to Claim 12 or 13, characterised in that the support member (23) has an elongated shape and comprises two rows of ampoules (1) disposed in one plane, on either side of the support member (23).

19. Cluster of ampoules (1) according to one of Claims 12 to 18, characterised in that the support member (23; 61) is arranged in order to be able to be connected to a customary needle (29).

20. Cluster of ampoules (1) according to one of Claims 12 to 19, characterised in that the ampoules (1) comprise a cylindrical body (2) defining an inner volume (8) and a piston (9) adapted to move in a tight manner in said body (2), in order to vary the inner volume (8) and in that, when the piston (9) is in its maximum retracted position, the inner volume (8) is greater by a regulated value than the volume of the dose for which the ampoule (1) is intended.

21. Support member serving to connect single-dose ampoules for single use and to ensure a flow of liquid from and to these ampoules, comprising an elongated body (61) provided with a central channel (62) and collateral channels (63a) opening out outside and into the central channel, a central channel (62) in which is inserted a spindle (69) able to rotate in this channel, this spindle (69) comprising a groove (73) extending along the spindle (69) and over part of the length of the latter and arranged in order to be able to arrive opposite collateral channels (63a) for their connection, and a groove (72), whereas the support member (61) comprises an outlet orifice (67) opening into the central channel (62) and facing the groove (72).

22. Support member according to Claim 21, characterised in that the elongated body (61) is made of flexible plastics material and associated with a rigid reinforcement (68).

23. Support member according to Claim 21 or 22, characterised in that the elongated body (61) comprises receptacles into which the collateral channels open and which are adapted to the ampoules (1).

24. Apparatus for the distribution of a pharmaceutical solution, contained in a multi-dose bottle, into single-dose ampoules for single use, in particular for dissolving a lyophilised pharmaceutical product and for the distribution of the pharmaceutical solution obtained, comprising a housing (31; 76) defined on at least one side by a structure (33; 80) which is fixed or movable in the plane of the housing (31) and by pushing rods (46) which pass through the structure (33; 80) through appropriate equidistant bores (40) and are movable in the plane of the housing (31; 76) by sliding in the bores (40).

25. Apparatus according to Claim 24, characterised in that, at its end situated adjacent the housing (31; 76), each pushing rod (46) comprises form-locking engagement means (48).

26. Apparatus according to Claim 25, characterised in that the engagement means comprise a lug (48) provided with a terminal head connected to the pushing rod (46) by a part of smaller section.

27. Apparatus according to one of Claims 24 to 26, characterised in that, associated with each bore (40) in the structure (33; 80) is a calibrated annular rim (41) which projects inside the housing (31).

28. Method for dissolving a lyophilised pharmaceutical product contained in a multi-dose bottle and its distribution into single-dose ampoules for single use, in particular for injection by pressurised jet, in which one uses single-dose ampoules containing the solvent adapted to the lyophilised product and connected to the multi-dose bottle by a network of channels, the solvent contained in all the ampoules is simultaneously passed into the bottle of lyophilised product, it is shaken in order to dissolve the product, then the pharmaceutical solution produced in this way is distributed in a metered manner simultaneously into all the single-dose ampoules, the solvent and pharmaceutical solution circulating in the network of channels by delivery and suction.

29. Method according to Claim 28, characterised in that initially one chooses single-dose ampoules containing excess solvent in order to take into account losses of solvent during the process.

## Patentansprüche

1. Vorrichtung zum Verteilen einer pharmazeutischen Lösung, die in einem Flakon für mehrere Dosen enthalten ist, in Ampullen für Einzeldosen für einmalige Verwendung, insbesondere für die Lösung eines lyophilisierten pharmazeutischen Produktes, das in einem solchen Flakon enthalten ist, und für die Verteilung der erhaltenen pharmazeutischen Lösung, bestehend aus Ampullen für Einzeldosen (1) und einem Verteilungsgerät (30; 74), wobei bei der Vorrichtung Förder-/Ansaugmittel (9, 46), die ausgeführt sind, um eine Flüssigkeit von den Ampullen (1) zu dem Flakon (60) und von dem Flakon (60) zu den Ampullen (1) mit Hilfe eines geeigneten Kanalnetzes (26, 27; 62, 67, 72, 73) zirkulieren lassen zu können, und eventuell ein Flakon (60) mit einem flüssigen oder lyophilisierten pharmazeutischen Produkt vorgesehen sind.

2. Vorrichtung nach Anspruch 1, dadurch gekennzeichnet, daß sie Mittel zur Regelung des Volumens der in die Ampullen (1) gefüllten pharmazeutischen Lösung umfaßt.

3. Vorrichtung nach Anspruch 1 oder 2, dadurch gekennzeichnet, daß die Ampullen für Einzeldosen (1) von einem zylindrischen Körper (2) gebildet werden, der ein Innenvolumen (8) begrenzt, in dem auf dichte Weise ein Kolben (9) gleitet, der derart ausgeführt ist, daß er das Innenvolumen (8) verändern kann, wobei die Kolben (9) der Ampullen für Einzeldosen (1) als Förder-/Ansaugmittel dienen, um die Flüssigkeit von den Ampullen (1) zu dem Flakon (60) und umgekehrt zirkulieren zu lassen.

4. Vorrichtung nach Anspruch 3, dadurch gekennzeichnet, daß der zylindrische Körper (2) der Ampullen für Einzeldosen (1) an einem Ende (4) durch einen Hals (6) verschlossen ist, der eine Mittelöffnung (7) aufweist, die eine Düse für Druckstrahleinspritzung bildet, wobei der Kolben (9) auch ausgeführt ist, um die Bildung dieses Strahls zu gewährleisten.

5. Vorrichtung nach einem der Ansprüche 1 bis 4, dadurch gekennzeichnet, daß die Ampullen (1) in einer Gruppe zu mehreren vorhanden sind, indem sie dicht und abnehmbar auf einem Stützteil (23; 61) montiert sind, der innen einen Mittelkanal (26; 62) aufweist, der über kollaterale Kanäle (27; 63a) jede der Ampullen für Einzeldosen (1) der Gruppe versorgt, wobei der Mittelkanal (26; 62) durch einen Teil (28; 66) des Stützteils (23; 61) mündet, der derart angeordnet ist, daß der Mittelkanal (26; 62) an das Innenvolumen eines Flakons für mehrere Dosen (60) angeschlossen werden kann.

6. Vorrichtung nach einem der Ansprüche 2 bis 5, dadurch gekennzeichnet, daß das Verteilungsgerät (30; 74) eine Lagerung (31; 76), in der die Ampullen für Einzeldosen (1) abnehmbar angeordnet sind, Schubstangen (46), die jeweils derart angeordnet sind, daß sie in lösbaren Eingriff mit dem Kolben (9) einer Ampulle (1) kommen können, und Betätigungsmittel (49, 51; 77, 78) umfaßt, um die Schubstangen (46) und folglich die Kolben (9) der Ampullen (1) in die Richtung der Verringerung des Innenvolumens (8) derselben oder in die umgekehrte Richtung zu verschieben, wobei ferner Mittel (41) vorgesehen sind, um in letztgenanntem Fall die Freigabe des Eingriffs zwischen Kolben (9) und Schubstangen (46) zu gewährleisten, so daß jede Ampulle (1) nun das erforderliche Volumen an pharmazeutischer Lösung enthält.

7. Vorrichtung nach Anspruch 6, dadurch gekennzeichnet, daß die Mittel zur Freigabe des Eingriffs zwischen Kolben (9) und Schubstangen (46) kalibrierte ringförmige Teile (41) sind, die von dem Gerät (30; 74) getragen werden und deren Außendurchmesser etwas kleiner als der Innendurchmesser des Körpers (2) der Ampulle (1) ist, wobei die ringförmigen Teile (41) derart ausgeführt sind, daß sie sich im Inneren des Ampullenkörpers (2) befinden, so daß sie einen Anschlag für den Kolben (9) der Ampulle (1) bei seiner Verschiebung in die Richtung der Vergrößerung des Innenvolumens (8) der Ampulle (1) bilden.

8. Vorrichtung nach einem der Ansprüche 5 bis 7, dadurch gekennzeichnet, daß der Stützteil (61) die Ampullen (1) auf einer einzigen und selben Seite (64) aufweist.

9. Vorrichtung nach einem der Ansprüche 5 bis 7, dadurch gekennzeichnet, daß der Stützteil (23) die Ampullen (1) auf zwei gegenüberliegenden Seiten aufweist.

10. Vorrichtung nach einem der Ansprüche 6 bis 9, dadurch gekennzeichnet, daß jede Schubstange (46) an einem Ende mit einem Eingriffsmittel (48) versehen ist, das zu einem Eingriffsmittel (15) komplementär ist, das auf den Kolben (9) der Ampullen (1) vorhanden ist.

11. Vorrichtung nach einem der Ansprüche 1 bis 10, dadurch gekennzeichnet, daß sie ein Bündel von Ampullen (1) nach einem der Ansprüche 12 bis 21 und/oder einen Stützteil nach einem der Ansprüche 22 bis 24 und/oder ein Verteilungsgerät nach einem der Ansprüche 25 bis 28 umfaßt.

12. Bündel von Ampullen für Einzeldosen (1) für einmalige Verwendung, die für das Einspritzen einer pharmazeutischen Lösung durch Druckstrahl bestimmt sind und auf einem Stützteil (23; 61) vereint sind, der innen ein Kanalnetz (26, 27; 62, 63a, 67, 72, 73) umfaßt, das zum Versorgen der Ampullen (1) angeordnet ist.

13. Bündel von Ampullen (1) nach Anspruch 12, dadurch gekennzeichnet, daß die Ampullen (1) ein Ende (4) umfassen, das eine Endöffnung aufweist und eine Einspritzdüse (7) bildet, daß der Stützteil (23; 61) Behälter (22; 63) umfaßt, in denen die Ampullenenden, die Einspritzdüsen (7) bilden, dicht und abnehmbar angeordnet sind, und daß das Kanalnetz des Stützteils (23; 61) jeweils einen kollateralen Kanal (27; 63a) umfaßt, der in den Behälter (22; 63) gegenüber der Endöffnung der Einspritzdüse (7) der vorhandenen Ampulle (1) mündet.

14. Bündel von Ampullen (1) nach Anspruch 12 oder 13, dadurch gekennzeichnet, daß der Stützteil (61) die Ampullen (1) auf einer selben Seite (64) umfaßt.

15. Bündel von Ampullen (1) nach Anspruch 14, dadurch gekennzeichnet, daß der Stützteil (61) einen Mittelkanal (62) umfaßt, in den eine in diesem Kanal drehbare Achse (69) eingesetzt ist, wobei diese Achse (69) mit einer Nut (73), die sich entlang der Achse (69) und auf einem Teil der Länge derselben erstreckt, wobei die Nut (73) derart angeordnet ist, daß sie die Ampullen (1) miteinander in Verbindung bringen kann oder diese Verbindung unterbrechen kann, und mit einer Rille (72) versehen ist, während der Stützteil (61) eine Ausgangsöffnung (67) umfaßt, die in den Mittelkanal (62) gegenüber der Rille (72) mündet.

16. Bündel von Ampullen (1) nach Anspruch 15, dadurch gekennzeichnet, daß die Ampullen (1) in einer Reihe angeordnet sind.

17. Bündel von Ampullen (1) nach einem der Ansprüche 14 bis 16, dadurch gekennzeichnet, daß der Stützteil (61) aus einem biegsamen Material besteht und mit einer starren Verstärkung (68) verbunden ist.

18. Bündel (21) von Ampullen (1) nach Anspruch 12 oder 13, dadurch gekennzeichnet, daß der Stützteil (23) eine längsförmige Form aufweist und zwei Reihen von Ampullen (1) umfaßt, die in einer Ebene auf beiden Seiten des Stützteils (23) angeordnet sind.

19. Bündel von Ampullen (1) nach einem der Ansprüche 12 bis 18, dadurch gekennzeichnet, daß der Stützteil (23; 61) derart angeordnet ist, daß er mit einer üblichen Nadel (29) verbunden werden kann.

20. Bündel von Ampullen (1) nach einem der Ansprüche 12 bis 19, dadurch gekennzeichnet, daß die Ampullen (1) einen zylindrischen Körper (2), der ein Innenvolumen (8) begrenzt, und einen Kolben (9) umfassen, der derart ausgeführt ist, daß er sich dicht in diesem Körper (2) verschieben kann, um das Innenvolumen (8) zu verändern, und daß das Innenvolumen (8), wenn sich der Kolben (9) in seiner maximal eingezogenen Position befindet, größer als ein Wert ist, der für das Volumen der Dosis, für die die Ampulle (1) bestimmt ist, eingestellt ist.

21. Stützteil, der zur Vereinigung der Ampullen für Einzeldosen für einmalige Verwendung und zur Gewährleistung eines Fließens von Flüssigkeit von und zu diesen Ampullen dient, bestehend aus einem längsförmigen Körper (61), der mit einem Mittelkanal (62) und kollateralen Kanälen (63a) versehen ist, die nach außen und in den Mittelkanal münden, wobei in den Mittelkanal (62) eine in diesem Kanal drehbare Achse (69) eingesetzt ist, wobei diese Achse (69) eine Nut (73), die sich entlang der Achse (69) und auf einem Teil der Länge derselben erstreckt und derart angeordnet ist, daß sie den kollateralen Kanälen (63a) für deren Verbindung gegenüberliegen kann, und eine Rille (72) umfaßt, während der Stützteil (61) eine Ausgangsöffnung (67) umfaßt, die in den Mittelkanal (62) gegenüber der Rille (72) mündet.

22. Stützteil nach Anspruch 21, dadurch gekennzeichnet, daß der längsförmige Körper (61) aus biegsamem Kunststoff besteht und mit einer starren Verstärkung (68) verbunden ist.

23. Stützteil nach Anspruch 21 oder 22, dadurch gekennzeichnet, daß der längsförmige Körper (61) Behälter umfaßt, in die die kollateralen Kanäle münden, und die an die Ampullen (1) angepaßt sind.

24. Gerät zum Verteilen einer pharmazeutischen Lösung, die in einem Flakon für mehrere Dosen enthalten ist, in Ampullen für Einzeldosen für einmalige Verwendung, insbesondere für die Lösung eines lyophilisierten pharmazeutischen Produktes und die Verteilung der erhaltenen pharmazeutischen Lösung, umfassend eine Lagerung (31; 76), die auf mindestens einer Seite von einer Struktur (33; 80), die fest oder in der Ebene der Lagerung (31) beweglich ist, und von Schubstangen (46) begrenzt ist, die durch die Struktur (33; 80) durch in gleichem Abstand angeordnete geeignete Bohrungen (40) hindurchgehen und in der Ebene der Lagerung (31; 76) durch Gleiten in den Bohrungen (40) beweglich sind.

25. Gerät nach Anspruch 24, dadurch gekennzeichnet, daß an dem Ende, das sich auf der Seite der Lagerung (31; 76) befindet, jede Schubstange (46) ein Mittel (48) zum Eingriff durch Formsperre umfaßt.

26. Gerät nach Anspruch 25, dadurch gekennzeichnet, daß das Eingriffemittel eine Spitze (48) umfaßt, die mit einem Endkopf versehen ist, der mit der Schubstange (46) durch einen Teil kleineren Querschnitts verbunden ist.

27. Gerät nach einem der Ansprüche 24 bis 26, dadurch gekennzeichnet, daß es an jeder Bohrung (40) der Struktur (33; 80) mit einer ringförmigen kalibrierten Leiste (41) verbunden ist, die im Inneren der Lagerung (31) vorspringt.

28. Verfahren zur Lösung eines lyophilisierten pharmazeutischen Produktes, das in einem Flakon für mehrere Dosen enthalten ist, und zu dessen Verteilung in Ampullen für Einzeldosen für einmalige Verwendung, insbesondere für das Einspritzen unter Druckstrahl, bei dem Ampullen für Einzeldosen verwendet werden, die das entsprechende Lösungsmittel für das lyophilisierte Produkte enthalten und mit dem Flakon für mehrere Dosen durch ein Kanalnetz verbunden sind, bei dem gleichzeitig das Lösungsmittel, das in der Gesamtheit der Ampullen vorhanden ist, in den Flakon des lyophilisierten Produktes eingebracht wird, bei dem das Produkt für ein gutes Lösen geschüttelt wird, sodann die auf diese Weise gleichzeitig in der Gesamtheit und bei dem der Ampullen für Einzeldosen erzeugte pharmazeutische Lösung dosiert verteilt wird, wobei das Lösungsmittel und die pharmazeutische Lösung in dem Kanalnetz durch Förderung und Ansaugung zirkulieren.

29. Verfahren nach Anspruch 28, dadurch gekennzeichnet, daß zu Beginn Ampullen für Einzeldosen gewählt werden, die überschüssiges Lösungsmittel enthalten, um die Lösungsmittelverluste während des Verfahrens zu berücksichtigen.
